Europäisches Patentamt

(19) European Patent Office

Office européen des brevets

(11) EP 0 916 677 A1

(12) **EUROPEAN PATENT APPLICATION**
published in accordance with Art. 158(3) EPC

(43) Date of publication:
19.05.1999 Bulletin 1999/20

(21) Application number: 97932982.8

(22) Date of filing: 23.07.1997

(51) Int. Cl.$^6$: **C07K 1/00**, G06F 15/18

(86) International application number:
PCT/JP97/02535

(87) International publication number:
WO 98/04580 (05.02.1998 Gazette 1998/05)

(84) Designated Contracting States:
AT BE CH DE DK ES FI FR GB GR IE IT LI LU NL
PT SE

(30) Priority: 26.07.1996 JP 198096/96

(71) Applicants:
• YAMANOUCHI PHARMACEUTICAL CO., LTD.
Tokyo 103 (JP)
• Karube, Masao
Kawasaki-shi, Kanagawa 216 (JP)

(72) Inventors:
• KARUBE, Masao
Kawasaki-shi, Kanagawa 216 (JP)
• YOKOBAYASHI, Youhei
Yokohama-shi, Kanagawa 241 (JP)

(74) Representative: HOFFMANN - EITLE
Patent- und Rechtsanwälte
Arabellastrasse 4
81925 München (DE)

(54) **METHOD FOR SEARCHING FOR PEPTIDES AND PROCESS FOR PRODUCING COMPOUNDS**

(57) Peptides having a higher physiological activity are screened by the steps of (a) synthesizing an optional number of peptides having different arbitrary amino acid sequences; (b) assaying the peptides synthesized in step (a) for their physiological activities to select an optional number of peptides having higher physiological activities; (c) synthesizing peptides having amino acid sequences obtained by genetic algorithm processing of the amino acid sequences of the peptides selected in step (b); and, (d) repeating step (b) and step (c), an optional number of times, for the peptides obtained in step (c). In genetic algorithms, each amino acid is represented by one notation and amino acid sequence of a peptide by the representation is used as a genome.

FIG.1

## Description

Technical Field

The present invention relates to a method for exploring a peptide having a higher physiological activity by selecting the amino acid sequence of a peptide in terms of its genetic algorithm and to a process for producing a compound using the peptide, which can achieve a higher physiological activity.

Background Art

Peptides or proteins exert diverse physiological activities depending upon their amino acid sequences. If such amino acid sequences of these peptides or proteins having desirable physiological activities are efficiently selected, such a selection technique will have extensive applications to wide areas including the fields of medical or food industries.

Those attempts have been hitherto made through expression of a protein corresponding to a given gene, using a phage expression system or by synthesizing a peptide having a given amino acid sequence by solid-phase synthesis.

These methodologies are to explore peptides, etc. having desirable physiological activities by preparing peptides suspected to have amino acid sequences with target physiological activities and assaying activities for these peptides.

However, in view of a large number of amino acid sequences required (for example, $20^{10}$, namely, $10^{13}$ amino acid sequences exist for even a peptide of 10 amino acids in which each residue is selected from 20 kinds of amino acids), there is a limit to the number of peptides to be prepared.

Moreover, these methodologies are exploration by trial and error to find a peptide having a desired physiological activity and thus labor-extensive and time-consuming, which made it difficult to reach a target peptide efficiently.

On the other hand, living organisms have acquired highly physiologically active proteins consisting of several hundreds of amino acids by exploiting a unique mechanism of evolution. It is known that genetic recombination plays a significant role for evolution, in addition to repeated selection and mutation.

Recently, an approach to use a mathematically arranged genetic algorithm (hereinafter abbreviated as GA) model to the mechanism of such evolution was proposed to explore a peptide having a desired physiological activity in a logical and efficient manner.

This approach is used to find amino acid sequences of peptides by applying operation corresponding to "selection", "genetic recombination" and "mutation", just like in the evolution of living organisms, to explore peptides having a high physiological activity.

Specifically, GA-based exploration is implemented by the following steps for operation.

(1) A population of peptides having a higher physiological activity is selected from peptides having arbitrary amino acid sequences (hereinafter this operation is called "selection");
(2) Next, each pair is chosen from all of the selected amino acid sequences are chosen to form pairs of amino acid sequences, which are then crossed-over, that is, exchange of the sequences in part (hereinafter this operation is called "genetic recombination").
(3) In the selected population of peptides, some amino acids in the sequence are replaced by other amino acids in a given mutation rate (hereinafter this operation is called "mutation").

The foregoing operations are implemented to bring close to optimization gradually.

The thus obtained population of peptides having new amino acid sequences is repeatedly subjected to the foregoing steps, whereby peptides having a higher physiological activity can be obtained as the generation is renewed to go forward.

Attempts to select peptides using GA are known by the method of Weber et al. (Angew. Chem. Int. Engl., 1995, 34, 2280) and the method of Singh et al. (J. Am. Chem. Soc., 1996, 118, 1669).

GA generally adopts to represent each member of amino acids by a binary bit string. For this reason, each of amino acids which consist peptides is also represented by the binary system in the above method. This representation encounters the following problems.

In any of the above methodologies, each amino acid can code for each of the binary sequence of 5 bits. It is then possible to represent 5 bits = $2^5$, namely, 32 amino acids at maximum. However, in the method of Singh et al., only 20 essential amino acids are represented by the 5 bits and adequate amino acids are arbitrarily represented by the remaining bits for 12 other amino acids. Accordingly, the 20 amino acids are not handled equivalently.

Therefore, sequences for the particular amino acids arbitrarily encoded are predominantly selected in exploring a peptide having a higher physiological activity. That is, exploration lacks its accuracy due to a biased mutation rate occurred in the selection process of amino acids.

In addition, these methods concede to use as a crossing point the internal position of each sequence in the binary system by which one amino acid is represented.

That is, the binary notation to represent one amino acid is intersected to cross at a random position.

In this case, it is quite unlikely that the intersected part of the amino acid corresponding to the binary notation be represented by the same amino acid notation; in most cases, the intersected part is replaced by other amino acid notation, that is, the same operation as "mutation" is carried out.

Furthermore, since the intersecting position is arbitrarily chosen so that apparently, the internal position of the binary system is chosen more frequently than the position between the binary system for representing one amino acid and that for another amino acid.

Therefore, in almost all cases, "mutation" occurs concurrently with "genetic recombination".

On the other hand, a probability that "mutation" occurs in operation is preset at a certain low rate in those methods. However, the "mutation" does not include the above "mutation" accompanied by "genetic recombination".

As this is the case, the "mutation" will occur at a considerably high rate in any of those methods, as compared to the given rate, which in turn leads to such problems that GA is departed from its intended purpose of exploring a target protein and efficiency of the exploration decreases.

Disclosure of Invention

The present invention has been made in view of the foregoing problems. An object of the present invention is to provide a method for exploring a peptide having a desired physiological activity accurately and efficiently by preventing a bias in probability for the amino acid selection and "mutation" accompanied by "genetic recombination". Another object of the present invention is to provide a process for producing a compound with a higher physiological activity, using such a peptide.

That is, according to the present invention, there is provided a method for exploring a peptide with a higher physiological activity which comprises the steps of:

(a): synthesizing an optional number of peptides having different arbitrary amino acid sequences;
(b): assaying the peptides synthesized in step (a) for their physiological activities to select an optional number of peptides having higher physiological activities;
(c): synthesizing peptides having amino acid sequences obtained by GA processing of the amino acid sequences of the peptides selected in step (b); and,
(d): repeating step (b) and step (c), an optional number of times, for the peptides obtained in step (c);

and which method is characterized in that each of amino acids to constitute peptides is represented by one notation and the amino acid sequences to construct the peptide represented by the notation are processed as genome.

According to the present invention, there is further provided a process for producing a compound having a higher physiological activity which comprises synthesizing the peptides explored in step (d) above and preparing the compound using the peptides as synthesis blocks.

Brief Description of the Drawings

Fig. 1 is a scheme for selecting the peptides of the present invention.
Fig. 2 illustratively shows the method for selection of the peptides for the next generation according to the present invention.
Fig. 3 is a flow chart showing the method for exploring peptides in accordance with the present invention.
Fig. 4 is a graph showing trypsin inhibitory activity of the peptide selected by the present invention for each particular generation.

Best Mode for Carrying Out the Invention

In the method of the present invention for exploring peptides, each of the amino acids to constitute peptides is distinctly represented by one notation and the thus represented amino acid sequence is processed as "genome". Thus, amino acids can be operated equivalently.

Therefore, the bias problem in probability inherently occurred in the amino acid selection step in the prior art methods can be obviated, which enables to conduct operation more accurately.

Moreover, "mutation" which occurs during "genetic recombination" operation can be controlled so that any decrease in exploration efficiency accompanied by an increased "mutation" rate can be prevented to provide efficient exploration of peptides.

To assign one notation to each of the amino acids which constitute peptides, one letter notation system using one of the alphabet can be used to represent each amino acid.

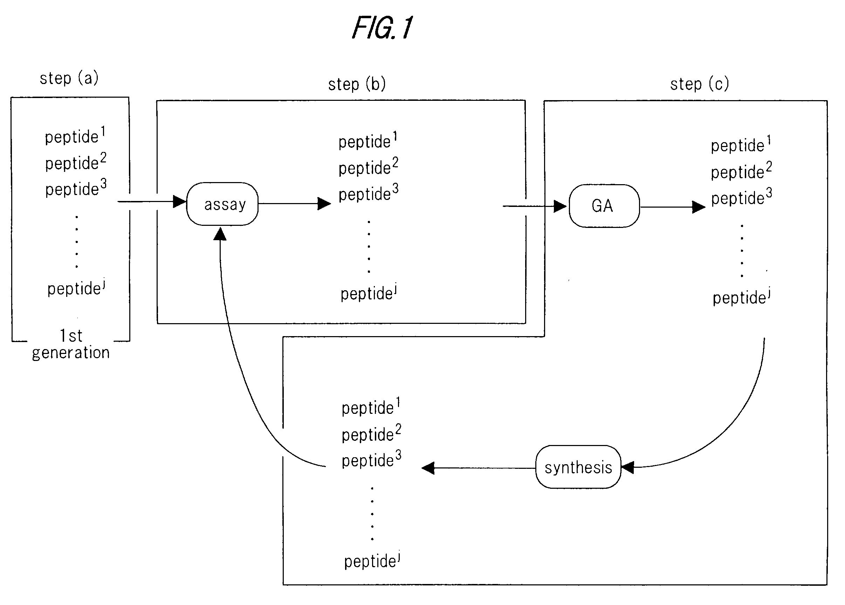

The method of the present invention for screening peptides is illustratively shown in Fig. 1. According to the screening method of the present invention, an optional number "i" of peptides having different arbitrary amino acid sequences (first generation) are synthesized instep (a). The term "peptide" refers to a compound produced by forming amide bonds between the same or different two or more amino acids. In the present invention, peptides of 5 to 20 amino acid residues are preferably employed.

In step (b), the peptides synthesized in step (a) are assayed for a desired physiological activity to select an optional number of peptides showing a higher physiological activity. The step of selecting peptides with a higher physiological activity corresponds to "selection" in the mechanism of evolution In the present invention, the term "physiological activity" is used to mean all functions that peptides can exhibit in vivo, such as enzyme inhibitory activity, catalytic activity, molecular recognizing ability, etc.

In step (c), peptides having the amino acid sequences of the peptides selected in step (b) are processed by GA and peptides having the GA-processed amino acid sequences are synthesized.

The amino acid sequences of the peptides remained after "selection" in step (b) are randomly grouped in pairs of two amino acid sequences. The "genetic recombination" is performed between the two amino acid sequences which constitute the respective pairs. The intersection for the genetic recombination is chosen at random and its frequency is preset as in the natural world. The amino acid sequences of each peptide is replaced by other amino acids in a probability of 3%, which corresponds to "mutation" in the mechanism of evolution. The preset frequency of 3% has been chosen to fit frequency of mutation occurred in the natural world. The "genetic recombination" may be performed prior to the "mutation" and vice versa.

The thus obtained peptides having the second generation amino acid sequences have a higher and lower physiological activity than those with the first generation amino acid sequences. When the entire generations are compared as a whole, however, the peptides of the second generation are higher in the physiological activity. This is because the amino acid sequences of the second generation are based on "genetic pool" which shows a higher physiological activity in the amino acid sequences of the first generation.

The peptide synthesis in step (a) and step (c) of the present invention there is carried out by known methods using Fmoc, t-Boc, etc., using a peptide synthesizer.

Step (b)and step (c) are repeated for the peptides having the amino acid sequences of the second generation. It is thus possible to explore peptides having a higher physiological activity as the generation proceeds from the second to the third and the fourth generations.

The present invention will be described in more detail with reference to the examples below but is not deemed to be limited thereto.

Example 1

A peptide having an activity of inhibiting trypsin, which is known to be a proteolytic enzyme, was selected by the method of the present invention. The peptide was a hexapeptide (a peptide of 6 amino acids) consisting of four amino acids of phenylalanine (F), isoleucine (I), lysine (K) and threonine (T), in which the N terminus was acetylated and the C terminus was amidated.

First, 24 amino acid sequences as the hexapeptide consisting the four amino acids were randomly created with a computer and, peptides of the first generation which had the created amino acid sequences were synthesized using a peptide synthesizer. The peptides were synthesized by the Fmoc process using a peptide synthesizer. After lyophilization, the peptides synthesized were analyzed for purity on reverse phase HPLC using MALDI-TOF/MS$^2$. When the peptides contained many impurities, they were purified by HPLC.

Next, the synthesized 24 hexapeptides (hereinafter simply referred to as peptides) were analyzed for the trypsin inhibitory activity. A change in degradation of BAPA (below) with trypsin in the presence of the peptides described above was determined by monitoring a change in absorbance. A change from the initial reaction rate was expressed in percentage, which was made the trypsin inhibitory activity.

BAPA: N$^\alpha$-benzoyl-DL-arginine-p-nitroanilide

More specifically, 400 $\mu$l of 0.1M Tris/HCl buffer (pH 7.8), 200 $\mu$l of 1 mM peptide Tris buffer (pH 7.8) solution, 400 $\mu$l of 2.3 mM BAPA 10% DMSO solution and 60 $\mu$l of 1 mg/ml trypsin 0.02N HCl solution were mixed in a cuvette. Absorbance of the mixture was measured at 410 nm every 5 seconds for 3 minutes. On a graph where time was plotted on the abscissa and absorbance on the ordinate, a slope was formed between 1 and 3 minutes after the initiation of monitoring, which was made Δp. The same run was performed except that no peptide was added and the thus obtained slope was made Δb. A trypsin inhibitory activity was calculated in terms of a relative inhibitory activity according to the following equation:

$$100 \times (\Delta b - \Delta p)/\Delta b$$

From the 24 peptides described above, 18 peptides were selected in the order of higher activity. The remaining peptides were discarded for "selection". In the peptides showing the highest to sixth highest activity, the same two members were selected and 24 peptides in total were kept as the parents for the second generation.

Next, the amino acid sequences of the selected peptides were subjected to "genetic recombination" and "mutation" using GA. The application of GA was computerized using a commercially available software (e.g., TURBO PASCAL, trade name).

Two amino acids from the 24 peptides selected by the procedure above were randomly put together to form 12 pairs. From five possible intersections formed by six amino acids one intersection was arbitrarily chosen for "genetic recombination".

Next, each amino acid in each peptide was replaced with other amino acid in a probability of 3% to induce "mutation".

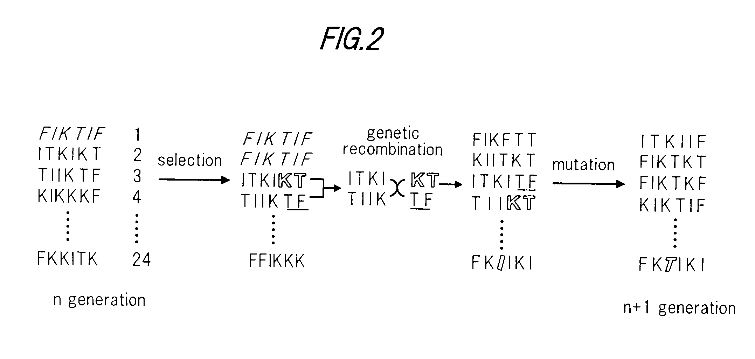

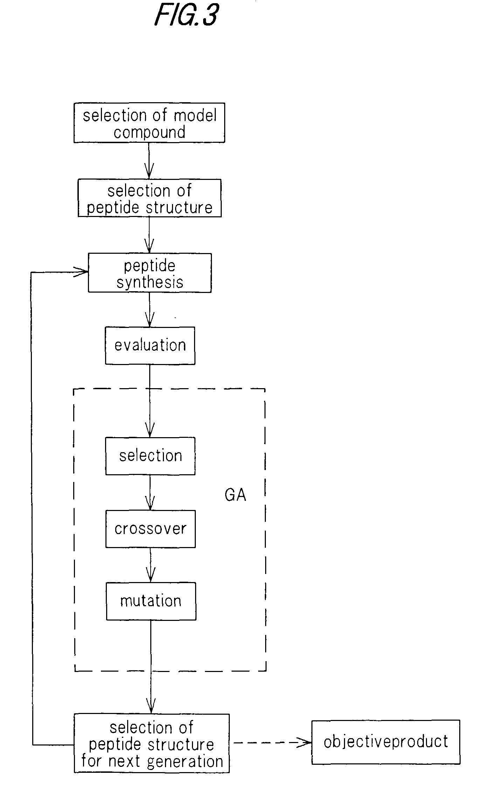

Fig. 2 illustratively shows the procedures performed in this Example, which correspond to "selection", "genetic recombination" and "mutation". Fig. 3 is a flow chart showing each procedure of this Example 1 described above.

The peptides of the second generation having the amino acid sequences thus obtained were synthesized. After measurement of the trypsin inhibitory activity for each peptide, "selection", "genetic recombination" and "mutation" were applied to the peptides in a similar manner to those of the first generation. Thus, the peptides of the third generation were obtained. By repeating these steps, the peptides up to the sixth generation were prepared.

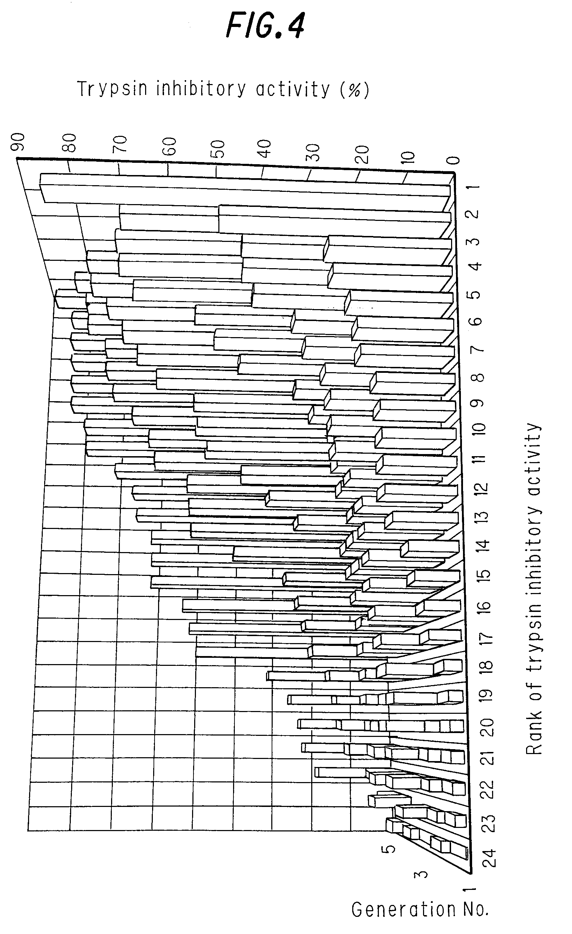

Fig. 4 shows the peptides from the first to sixth generations, in which the peptides are ranked by the highest trypsin inhibitory activity. It is noted from Fig. 4 that as the generation goes forward, the trypsin inhibitory activity increases as the entire generation.

A peptide Ac-TTKIFT-NH$_2$, which was discovered by Eichler et al. using a synthetic library, is known to have a high trypsin inhibitory activity. It is also known that a sequence of XXKIXX or XKIXXX (X is an optional amino acid) is important for trypsin inhibitory activity (Biochemistry, 1993, 32, 11035). In this Example, the peptides ranked the first to 35th throughout the entire generations had the sequence of XXKIXX or XKIXXX except that a peptide having a sequence of TTKIFT appeared in the sixth generation.

Throughout the first to sixth generations, 16 peptides were selected from all the peptides in the order of a higher trypsin inhibitory activity. The amino acid sequences of the selected peptides are shown in Table 1. In Table 1, when the generation number is shown by two or more in "Generation where sequences exist", it means that peptides with the same sequence appeared in the generations indicated. Where the same generation number is shown by two or more, it means that two or more of the same peptide appeared in the same generation.

Table 1

| Rank | Sequence | Trypsin inhibitory activity (%) | Generation where sequences exist |
|------|----------|--------------------------------|----------------------------------|
| 1 | TTKIFT | 89.0 | 6 |
| 2 | KKIFIF | 85.9 | 1 |
| 3 | TKIFKI | 84.3 | 6 |
| 4 | FTKIKI | 84.1 | 6 |
| 5 | KTKIKI | 83.6 | 6, 6 |
| 6 | FKKIFI | 83.2 | 6 |
| 7 | TTKIKI | 82.4 | 5 |
| 8 | FKKIFT | 79.9 | 6 |
| 9 | TKIIKI | 79.3 | 4, 5 |
| 10 | TTKIFI | 78.8 | 5 |

EP 0 916 677 A1

Table 1 (continued)

| Rank | Sequence | Trypsin inhibitory activity (%) | Generation where sequences exist |
|---|---|---|---|
| 11 | ITKIKI | 78.4 | 4, 6 |
| 12 | TKKIKI | 74.4 | 5 |
| 13 | FKKIKI | 74.0 | 5 |
| 14 | KTKIKT | 72.2 | 5 |
| 15 | TTKIKT | 72.1 | 3, 4, 6 |
| 16 | KKIIKI | 70.6 | 3 |

In the 16 peptides shown above, 9 peptides exist in the 6th generation, 6 in the fifth generation and 3 in the fourth generation. Thus, it is seen that a proportion of the peptides appeared in the later generation in the 16 peptides becomes larger.

Where one selects a peptide having TTKIFT sequence in a conventional manner, it will take approximately 27 weeks. When selected in accordance with the present invention, it took only about 18 weeks. It is noted that the shortened period enables to select peptides efficiently.

Industrial Applicability

According to the method for exploring peptides of the present invention, one notation is assigned, in GA, to each member of the amino acids which constitute peptides and hence, amino acids can be operated equivalently.

Therefore, the present invention can obviate the prior art problem that when amino acids are selected, there is a bias in a probability. Then, the operation can be made more accurately.

By controlling "mutation" occurred in the "genetic recombination" operation, a decrease in exploration efficiency accompanied by an increased "mutation" rate can be prevented, which in turn enables to explore peptides efficiently. Therefore, the time and labor required for exploration of peptides having a desired physiological activity can be greatly minimized.

The peptides thus explored and synthesized can be used as synthetic blocks, which enables to prepare more complicated compounds like proteins, enzymes, etc., having a higher physiological activity.

## Claims

1.    A method for exploring a peptide with a higher physiological activity which comprises the steps of:

   (a) synthesizing an optional number of peptides having different arbitrary amino acid sequences;
   (b) assaying the peptides synthesized in step (a) for their physiological activities to select an optional number of peptides having higher physiological activities;
   (c) synthesizing peptides having amino acid sequences obtained by genetic algorithm processing of the amino acid sequences of the peptides selected in step (b); and,
   (d) repeating step (b) and step (c), an optional number of times, for the peptides obtained in step (c);

   wherein each of the amino acids to constitute peptides is represented by one notation and the amino acid sequences to construct the peptide represented by the notation are processed as a genome.

2.    A method according to claim 1, wherein the amino acids to construct the amino acid sequence are in a range of 5 to 20.

3.    A method according to claim 1 or 2, wherein the amino acid sequences are replaced by other amino acids in a probability of 3% as amino acid sequence in genetic algorithm.

4.    A process for producing a compound having a higher physiological activity which comprises synthesizing peptides explored at step (d) of claim 1 and preparing the compound using the peptides as synthesis blocks.

6

## FIG.1

step (a)

peptide$^1$
peptide$^2$
peptide$^3$
$\vdots$
peptide$^j$

1st
generation

step (b)

peptide$^1$
peptide$^2$
peptide$^3$
$\vdots$
peptide$^j$

assay

step (c)

GA

peptide$^1$
peptide$^2$
peptide$^3$
$\vdots$
peptide$^j$

peptide$^1$
peptide$^2$
peptide$^3$
$\vdots$
peptide$^j$

synthesis

EP 0 916 677 A1

# FIG.2

FIKTIF 1
ITKIKT 2      selection
TIIKTF 3   ⟶
KIKKKF 4
⋮    ⋮
FKKITK 24

n generation

FIKTIF
FIKTIF
ITKI**KT**
TIIK<u>TF</u>
⋮
FFIKKK

genetic recombination

ITKI ⟩⟨ **KT**
TIIK    <u>TF</u>   ⟶

FIKFTT
KIITKT
ITKI<u>TF</u>
TII**KT**
⋮
FK/IKI

mutation
⟶

ITKIIF
FIKTKT
FIKTKF
KIKTIF
⋮
FK𝒯IKI

n+1 generation

EP 0 916 677 A1

## FIG.3

# FIG.4

Trypsin inhibitory activity (%)

Rank of trypsin inhibitory activity

Generation No.

| INTERNATIONAL SEARCH REPORT | International application No. |
|---|---|
| | PCT/JP97/02535 |

**A. CLASSIFICATION OF SUBJECT MATTER**

$Int. Cl^6$  C07K1/00, G06F15/18

According to International Patent Classification (IPC) or to both national classification and IPC

**B. FIELDS SEARCHED**

Minimum documentation searched (classification system followed by classification symbols)

$Int. Cl^6$  C07K1/00, G06F15/18

Documentation searched other than minimum documentation to the extent that such documents are included in the fields searched

Electronic data base consulted during the international search (name of data base and, where practicable, search terms used)

CAS ONLINE, JICST File on Science and Technology

**C. DOCUMENTS CONSIDERED TO BE RELEVANT**

| Category* | Citation of document, with indication, where appropriate, of the relevant passages | Relevant to claim No. |
|---|---|---|
| PX | YOKOBAYASHI Yohei et al., "Directed evolution of trypsin inhibiting peptides using a genetic algorithm", Journal of the Chemical Society, Perkin transactions. 1, October 1996, Vol. 20, p. 2435-2437 | 1 - 4 |
| A | SINGH Jasbir et al., "Application of Genetic Algorithms to Combinatorial Synthesis: A Computational Approach to Lead Identification and Lead Optimization", Journal of the American Chemical Society, February 1996, Vol. 118, No. 7, p. 1669-1676 | 1 - 4 |
| A | WEBER Lutz et al., "Optimization of the Biological Activity of Combinatorial Compound Libraries by a Genetic Algorithm", Angewandte Chemie. International Edition in English, 1995, Vol. 34, No. 20, p. 2280-2282 | 1 - 4 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| September 26, 1997 (26. 09. 97) | October 7, 1997 (07. 10. 97) |

| Name and mailing address of the ISA/ | Authorized officer |
|---|---|
| Japanese Patent Office | |
| Facsimile No. | Telephone No. |

Form PCT/ISA/210 (second sheet) (July 1992)